Europäisches Patentamt

European Patent Office

Office européen des brevets

(19)

(11) Numéro de publication: **0 006 060**
**A1**

(12)

# DEMANDE DE BREVET EUROPEEN

(21) Numéro de dépôt: 79400339.2

(22) Date de dépôt: 29.05.79

(51) Int. Cl.²: **A 61 K 35/78**

(30) Priorité: 31.05.78 GB 2561578

(43) Date de publication de la demande:
12.12.79 Bulletin 79/25

(84) Etats Contractants Désignés:
AT BE CH DE FR GB IT LU NL SE

(71) Demandeur: Société Anonyme dite: LABORATOIRES DEBAT
60 rue de Monceau
F-75008 Paris(FR)

(72) Inventeur: Debat, Jacques
3 rue du Pierrier
F-92210 Saint-Cloud(FR)

(72) Inventeur: Lemoine, Jean
7 Sente de la Portaille
F-92380 Garches(FR)

(72) Inventeur: Lier, Françoise née Gabillault
7 rue Hector Berlioz Domaine de la Bataille
F-78370 Plaisir(FR)

(74) Mandataire: Clisci, Serge et al,
CABINET BEAU DE LOMENIE 55 rue d'Amsterdam
F-75008 Paris(FR)

(54) Extrait d'Inula, son procédé de préparation et son utilisation en tant que médicament.

(57) La présente invention concerne un extrait d'*Inula* et plus particulièrement d'*Inula viscosa* et d'*Inula graveolens*.

Elle concerne également le procédé de préparation dudit extrait à partir d'n solvant choisi parmi les alcools, cétones, éthers, esters, hydrocarbures, hydrocarbures halogénés et leurs mélanges. Ledit extrait est utile en thérapeutique en tant qu'agent bactériostatique et fongistatique.

EP 0 006 060 A1

Croydon Printing Company Ltd.

Extrait d'Inula, son procédé de préparation et son utilisation
en tant que médicament.

La présente invention concerne un extrait d'Inula
et de plantes apparentées, le procédé de préparation de cet
extrait et son utilisation en thérapeutique, notamment en
tant qu'agent bactériostatique et fongistatique.

Plus précisemment, l'invention concerne un extrait
du genre Inula choisi parmi l'ensemble constitué par les
Inula viscosa et Inula graveolens.

Les plantes du genre Inula appartiennent à la
famille des Composacées . Elles ont été en particulier
décrites par G : GARNIER dans l'ouvrage "Ressources
Médicinales de la Flore Française" vol. 2, page 1358
(Vigot Frères ed., Paris, 1961).

Les composants chimiques des diverses espèces
d'Inula ont été décrits dans "Phytochemistry 17, 1165
(1978)".

L'Inula viscosa et l'Inula graveolens qui croissent dans le bassin méditérranéen sont des espèces
si voisines que l'on ne peut les distinguer l'une de l'autre
qu'à l'époque de la floraison.

Il a été décrit par G. CALDES et al.,dans
"Planta Medica 27, 72 - 76 (1975) que les extraits d'Inula
graveolens (obtenus par extraction à l'eau chaude et respectivement à l'eau froide, puis lyophilisation) testés en
tant qu'agents anti-bactériens vis à vis de Staphylococcus
aureus et Streptococcus faecium et en tant qu'agents antimalaria vis à vis de Plasmodium berghei, sont inactifs.

Il a été trouvé de façon surprenante, que l'extrait d'<u>Inula viscosa</u> et d'<u>Inula graveolens</u> sont utiles en thérapeutique dans le traitement des maladies infectieuses en tant qu'agent bactériostatique et fongistatique.

Selon la présente invention, la plante entière (c'est à dire les tiges, les feuilles, les sommités fleuries, les fruits et les racines) ou une partie de la plante, est extraite avec au moins un solvant. Le produit ainsi obtenu est purifié, si nécessaire, et recueilli selon une méthode connue en soi.

L'extrait est mis en oeuvre en utilisant par litre de solvant 30 à 150 g de plante broyée sèche.

Parmi les solvants utilisables, on peut notamment mentionner les alcools (tels qu'en les méthanol, éthanol, propanol et isopropanol, les cétones (tels qu'en les acétone, méthyléthylcétone, métylpropylcétone), les éthers (tels qu'en les éthers diméthylique, diéthylique et diisopropylique), les esters (tels que l'acétate d'éthyle), les hydrocarbures (tels que les pentane, hexane, cyclopentane, cyclohexane, éther de pétrole) et, les hydrocarbures halogénés (tels que les chloroforme et chlorure de méthylène) et leurs mélanges.

Le meilleur mode pour mettre en oeuvre l'invention, consiste à extraire une <u>Inula</u> choisie parmi l'ensemble constitué par les <u>Inula viscosa</u> et <u>Inula graveolens</u>, avec un solvant choisi parmi l'ensemble constitué par l'éther diéthylique, l'acétate d'éthyle, l'éthanol et le chloroforme, les solvants préférés étant l'éther diéthylique, l'acétate d'éthyle, l'éthanol. De préférence, l'extraction est effectuée à partir de la plante entière broyée et séchée à l'étuve à 37°C.

D'autres avantages et caractéristiques de l'invention seront mieux compris à la lecture qui va suivre d'exemples de préparations nullement limitatifs.

Exemple 1 : Extrait total d'Inula viscosa.

200 g de plante entière d'Inula viscosa sont séchés à l'étuve à 37°C, broyés et extraits avec 3 l d'éthanol dans un appareil Soxhlet pendant 4 h. On écarte l'insoluble et recueille la solution éthanolique que l'on sèche sur sulfate de sodium, et évapore à sec sous pression réduite. On obtient 8 g d'extrait mou.

Exemple 2 : Extrait total d'Inula viscosa En utilisant du chloroforme au lieu de l'éthanol et en procédant comme indiqué à l'exemple 1, on obtient 8 g d'extrait mou.

Exemple 3 : Extrait total d'Inula viscosa

200 g de plante entière d'Inula viscosa sont séchés à l'étuve à 37°C, broyés et extraits avec 3 l. d'éther diéthylique à 5 - 12°C pendant 48 h. On écarte l'insoluble et recueille la solution éthérée que l'on évapore à sec sous pression réduite. On obtient 6,3 g d'extrait mou (qui a reçu le numéro de code C.94). Rendement = 3,1 % en poids par rapport à la plante de départ.

Les extraits des exemples 1 à 3 donnent, par chromatographie sur couche mince, les trois mêmes taches, dans les conditions opératoires suivantes :

     support :               silice :

     phase mobile :       hexane - chloroforme - méthanol (3:9:1 v/v/v) ;

     révélateur            acide sulfurique - vanilline (1 g de vanilline pour 100 ml de $H_2SO_4$ concentré de densité 1,84) ;

Exemples 4 à 6 : Extraits totaux d'Inula graveolens.

En traitant 200 g de plante entière d'Inula graveolens qui a été séchée à l'étuve à 37°C et broyée, selon les procédés décrits aux exemples 1 à 3, on obtient respectivement trois extraits mous d'_Inula graveolens_ avec des rendements identiques à ceux des exemples 1 à 3.

Exemple 7 : Extrait total d'_Inula viscosa_

400 g de plante entière d'_Inula viscosa_ sont séchés à 37°C à l'étuve, broyés et extraits avec 3 l. d'éther diéthylique dans un appareil de Soxhlet. L'insoluble est écarté et la solution éthérée que l'on recueille est séchée sur sulfate de sodium anhydre et évaporée à sec sous pression réduite. On obtient 7 g d'extrait total (rendement 1,75 % en poids par rapport à la plante de départ).

Exemple 8 :

L'extrait total de l'exemple 7 soumis à une chromatographie sur colonne [la colonne étant chargée avec la silice ("Kieselgel 60" fabriqué par la Société MERCK & Co)] donne quatre fractions (Fl - F4) comme indiqué dans le tableau 1.

TABLEAU 1

| Eluant | Fraction | Rendement |
|---|---|---|
| Hexane - chlorure de méthylène (50 : 50, v/v) | F 1 | 0,25 % |
| Hexane - chlorure de méthylène (50 : 50, v/v) (b) | F 2 | 0,10 % |
| Chlorure de méthylène | F 3 | 0,10 % |
| Chlorure de méthylène méthanol (99 : 1,v/v) | F 4 | 0,12 % |

**0006060**

Notes :

(a) en poids, par rapport à la plante de départ

(b) il faut plus de temps pour éluer F2 que F1.

Les extraits des exemples 1 - 7 selon l'invention présentent une activité bactériostatique vis à vis des bactéries Gram (+) et Gram (-) et une activité fongistatique vis à vis des champignons. En particulier, l'extrait de l'exemple 3 présente les CMI (concentration minimale inhibitrice) donnés dans le tableau II.

TABLEAU II

| Microorganisme | CMI $(\mu g/ml)$ |
|---|---|
| Staphylococcus aureus Londres | 1 |
| Escherichia coli | 33 |
| Proteus | 33 |
| Aspergillus niger | 3 |

Selon l'invention, on préconise une composition thérapeutique renfermant, en association avec un excipient physiologiquement acceptable, une quantité pharmaceutiquement efficace d'un extrait selon l'invention. Une telle composition peut être administrée par voie orale, locale ou injectable.

REVENDICATIONS
-------------------------------

1.        Procédé pour la préparation d'un extrait d'Inula
utile en thérapeutique caractérisé en ce que l'on fait
appel à une espèce d'Inula choisie parmi l'ensemble constitué par les Inula viscosa et Inula graveolens, on extrait
la plante entière ou une partie de celle-ci avec un solvant
organique choisi parmi l'ensemble constitué par les alcools,
cétones, éthers, esters, hydrocarbures, hydrocarbures halogénés
et leurs mélanges.

2.        Procédé de préparation d'un extrait d'Inula
utile en thérapeutique en tant qu'agent bactériostatique
et fongistatique, caractérisé en ce que l'on soumet une
espèce d'Inula choisie parmi l'ensemble constitué par les
Inula viscosa et Inula graveolens, à une extraction avec au
moins un solvant organique choisi parmi l'ensemble constitué
par les alcools, cétones, éthers, esters, hydrocarbures, hydrocarbures halogénés et leurs mélanges, à raison de 1 l de
solvant organique pour 30 à 150 g de plante.

3.        Procédé selon la revendication 2 caractérisé en
ce que l'on extrait 30 à 150 g de plante entière broyée et
sèche avec 1 l d'un solvant organique choisi parmi l'ensemble constitué par l'éthanol, l'acétate d'éthyle, l'éther
diéthylique et le chloroforme, et en ce que l'on évapore à
sec la solution ainsi obtenue sous pression réduite.

4.        Procédé selon la revendication 2 caractérisé en
ce que l'on extrait 30 à 150 g d'une partie de la plante
broyée et sèche choisis parmi les tiges, les feuilles, les
sommités fleuries, les fruits et les racines, avec 1 l d'un
solvant organique choisi parmi l'ensemble constitué par
l'éthanol, l'acétate d'éthyle, l'éther diéthylique et le
chloroforme, et en ce que l'on évapore à sec la solution
ainsi obtenue sous pression réduite.

5.       Extrait d'_Inula_ caractérisé en ce qu'il est obtenu à partir d'une espèce choisie parmi l'ensemble constitué par les _Inula viscosa_ et _Inula graveolens_, selon le procédé de la revendication 3.

6.       Composition thérapeutique utile dans le traitement des êtres humains souffrant notamment de maladies infectieuses, caractérisée en ce qu'elle renferme, en association avec un excipient physiologiquement acceptable, une quantité pharmaceutiquement efficace d'un extrait d'_Inula_, selon la revendication 5.

**Office européen des brevets**

**RAPPORT DE RECHERCHE EUROPEENNE**

| DOCUMENTS CONSIDERES COMME PERTINENTS | | | CLASSEMENT DE LA DEMANDE (Int. Cl.³) |
|---|---|---|---|
| Catégorie | Citation du document avec indication, en cas de besoin, des parties pertinentes | Revendication concernée | |
| | CHEMISCHE BERICHTE, Vol. 110 (1977). Article de: F. BOHLMANN, H.CZERSON et S. SCHOENEWEISS: "Neue Inhaltsstoffe aus Inula viscosa Ait" pages 1330-1334 <br><br> * Pages 1333 et 1334, paragraphe "Isolierung der Inhaltsstoffe" * <br><br> ---- | 1,2 | A 61 K 35/78 |

**DOMAINES TECHNIQUES RECHERCHES (Int. Cl.³)**

A 61 K 35/78

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons
&: membre de la même famille, document correspondant

Le présent rapport de recherche a été établi pour toutes les revendications

| Lieu de la recherche | Date d'achèvement de la recherche | Examinateur |
|---|---|---|
| La Haye | 24-08-1979 | REMPP |

OEB Form 1503.1 06.78